# EUROPÄISCHE PATENTSCHRIFT

(11) **EP 0 938 316 B1**
(45) Veröffentlichungstag und Bekanntmachung des Hinweises auf die Patenterteilung: **24.07.2002**
(21) Anmeldenummer: 97954355.0
(22) Anmeldetag: 04.12.1997
(51) Int. Cl.: A61K 31/485, A61K 9/16, A61K 9/50

(54) **OPIOIDANTAGONISTHALTIGE GALENISCHE FORMULIERUNG**
GALENIC COMPOSITION CONTAINING OPIOID ANTAGONISTS
COMPOSITION GALENIQUE CONTENANT DES ANTAGONISTES DES OPIOIDES

(30) Priorität: 11.12.1996 DE 19651551
(43) Veröffentlichungstag der Anmeldung: 01.09.1999
(73) Patentinhaber: Norgine Europe BV, 1101 CA Amsterdam Zuid-Oost (NL)
(72) Erfinder: WALTER, Kersten, D-81549 München (DE); PROFITLICH, Thomas, D-81929 München (DE)
(74) Vertreter: Silveston, Judith
(86) Internationale Anmeldenummer: EP9706789
(87) Internationale Veröffentlichungsnummer: WO9825613

(56) Entgegenhaltungen:
- EP-A- 0 352 361
- WO-A-83/03197
- WO-A-97/33566
- DE-A- 4 325 465
- US-A- 3 966 940
- US-A- 4 774 230

## Beschreibung

Die Erfindung betrifft neue galenische Formulierungen mit opioidantagonistischer Aktivität und deren Verwendung bei der opioidinduzierten Obstipation. Insbesondere betrifft die Erfindung Pellet-, Granulat- oder Microtablettenformulierungen, die die Wirkstoffe Naloxon, N-Methylnaloxon oder N-Methylnaltrexon als Wirkstoffe mit opioidantagonistischer Aktivität enthalten.

Bei der Anwendung von stark wirksamen Analgetika vom Morphintyp stellt die durch Medikation auftretende Obstipation ein großes Problem dar. Sie gilt als eine der häufigsten Nebenwirkungen und ist besonders in der Dauertherapie eine unerwünschte Begleiterscheinung. Bei etwa 85 % der Patienten, denen Morphin gegeben wird, tritt dieses Problem während der Behandlung auf. Im Gegensatz zu anderen Nebenwirkungen, die durch z.B. Morphin hervorgerufen werden, handelt es sich um eine chronische Erscheinung, die im Laufe der Behandlung nicht an Intensität verliert [Saller R., Hellenbrecht D."Schmerzen-Therapie in Praxis und Klinik", 1. Auflage (1991), Marseille Verlag, München]. Die lähmende Wirkung von Opioiden auf die Darmmotilität ist schon lange bekannt und wird z.B. im Falle von Durchfallerkrankungen auch therapeutisch genutzt [Manara L. , Bianchetti A. "The central and peripheral influences of opioids on gastrointestinal propulsion", Ann. Rev. Pharmacol. Toxicol. 25, 249-273 (1985)]. Der Wirkungsmechanismus der Opioide auf die Darmmotilität ist zwar noch nicht vollständig aufgeklärt, wird aber im Zusammenhang mit der Bindung des Opioids an Opioidrezeptoren im Darm gesehen. Diese Opioidrezeptoren sind außer im Gehirn vor allem auch im Gastrointestinaltrakt in großer Dichte zu finden [Manara L. , Bianchetti A. "The central and peripheral influences of opioids on gastrointestinal propulsion", Ann. Rev. Pharmacol. Toxicol. 25, 249-273 (1985)].

In einer Reihe pharmakologischer Untersuchungen konnte gezeigt werden, daß Opioide (als Modellsubstanz wurde meist Morphin gewählt) eine direkte Wirkung auf die glatte Muskulatur des Darmes haben, und so der Muskeltonus in den Darmsegmenten zunimmt. Die Steigerung des segmentalen Tonus führt bei gleichzeitiger Abnahme der propulsiven Motilität des Darmes zu einer signifikanten Verlängerung der gastrointestinalen Passagezeit [Cameron J.C. "Constipation related to narcotic therapy", Cancer Nurs. 15, 372 377 (1992)] .

Ziel einer Therapie ist es, diese periphere Nebenwirkung des Morphins und verwandter Stoffe aufzuheben, da die opioidinduzierte Obstipation sehr schmerzhaft sein kann und schließlich den Behandlungserfolg gefährdet [Glare P., Lickiss J.N. "Unrecognized constipation in patients with advanced cancer: a recipe for therapeutic disaster", J. Pain Symptom Manage. 7, 369-371 (1992)]. Mit den üblichen Abführund Quellmitteln kann etwas mehr als die Hälfte der Patienten, die unter dieser Nebenwirkung leiden, ausreichend versorgt werden. Für den Rest der Patienten fehlen noch zufriedenstellende Behandlungsmöglichkeiten.

Da bei der opioidinduzierten Obstipation davon ausgegangen wird, daß die eigentliche Wirkung direkt und lokal über den gesamten Darm durch die Besetzung der Opioidrezeptoren abläuft, sollte dieser Effekt durch die Anwendung von Opioidantagonisten aufgehoben werden können. Die Verwendung von Opioidantagonisten ist allerdings nur dann sinnvoll, wenn sich die antagonistische Wirkung auf den Darm beschränkt und nicht die zentrale schmerzstillende Wirkung aufgehoben wird. Deshalb kommen nur wenige Opioidantagonisten wie z.B. Naloxon, N-Methylnaloxon oder N-Methylnaltrexon in Frage, die unter bestimmten Voraussetzungen peripher und nicht im ZNS wirken.

Naloxon ist ein reiner Opioidantagonist, der üblicherweise als Antidot im Vergiftungsfall mit Opioiden intravenös appliziert wird. Nach oraler Gabe wird Naloxon rasch und vollständig resorbiert. Da die Substanz einem sehr ausgeprägten First-Pass-Metabolismus unterliegt, sind nur geringe Mengen der unveränderten Substanz systemisch verfügbar. Der überwiegende Teil der applizierten Substanz liegt im Blut in Form der nicht oder nur schwach wirksamen Metaboliten Naloxon-3-Glukuronid, ß-Naloxol-3-Glukuronid und β-Naloxol vor [Vollmer K.O. "Pharmakokinetische Grundlagen des Valoron-N-Prinzips", Fortsch. Med. 106, 593-596 (1988)]. Aufgrund dieser pharmakokinetischen Eigenschaft ist Naloxon in geeigneter Dosis ein idealer Kandidat zur Behebung der opioidinduzierten Obstipation: im Darm liegt es als aktive Substanz vor und kann so die lähmende Wirkung des Opioids auf den Gastrointestinaltrakt aufheben, nach der Resorption wird es bei der ersten Leberpassage stark verstoffwechselt und unwirksam. Somit wird die schmerzstillende Wirkung der Opioide nicht beeinflußt.

In verschiedenen kleinen klinischen Studien konnte gezeigt werden, daß durch die orale Gabe von Naloxon die Opioidobstipation teilweise aufgehoben werden konnte: Basilisco et al. untersuchten in 2 Studien an gesunden Probanden den Einfluß von Loperamid, einem bei Durchfallerkrankungen eingesetzten peripher wirkenden Opioid, auf die gastrointestinale Passagezeit. Sie zeigten, daß Naloxon intravenös appliziert (40 µg/kg/Std. in 3 Std.) [Basilisco G., Bozzani A., Camboni G., Recchia M., Quatrini M., Conte D., Penagini R., Bianchi P.A. "Effect of loperamide and naloxone on mouth-to-caecum transit time evaluated by lactulose hydrogen breath test", Gut 26, 700-703 (1985)] und auch nach oraler Gabe von relativ hohen Dosen von 16 bzw. 32 mg die obstipierende Wirkung von Loperamid aufheben konnte [Basilisco G., Camboni G., Bozzani A., Paravicini M., Bianchi P.A. "Oral naloxone antagonizes loperamide-induced delay of orocecal transit", Dig. Dis. Sci. 32, 829-832 (1987)]. Culpepper-Morgan et al. berichten von einer Pilotstudie, in der 3 Patienten mit opioidinduzierter Obstipation mit oral appliziertem Naloxon behandelt wurden. Zwei der drei Patienten sprachen nach Dosen bis zu 16 mg auf die Behandlung an (Aufhebung der Obstipation). Bei dem weiteren Patienten konnte auch durch eine Dosissteigerung bis zu 24 mg Naloxon (innerhalb von 3 Stunden) die Obstipation nicht aufgehoben werden. Plasmaspiegelbestimmungen zeigten, daß dosisabhängig maximale Naloxonkonzentrationen bis zu 7,9 ng/ml gemessen wurden. Der Non-Responder zeigte bei einer Dosis ab 14 mg deutliche Entzugssymptome, die auf eine Antagonisierung der zentralen Opioidwirkung hinweisen [Culpepper-Morgan J.A., Inturrisi C.E., Portenoy R.K., Foley K., Houde R.W., Marsh F., Kreek M.J. "Treatment of opioid-induced constipation with oral naloxone: A pilot study", Clin. Pharmacol. Ther. 52, 90-95 (1992)].

Sykes berichtet von einer Studie mit 12 Patienten, denen orales Naloxon in unterschiedlichen Dosen verabreicht wurde. Die Naloxondosis orientierte sich an der täglichen Opioiddosis. Naloxon wurde in Dosen 0,5 %, 1 %, 2 %, 5 %, 10 %, 20 % und 40 %, bezogen auf die Opioiddosis, gegeben. Bis zur 10 % Naloxondosis war kein Effekt feststellbar. Erst im sehr hohen Dosisbereich (20% bis 40%) wird eine Aufhebung der Obstipation berichtet. Die absoluten Naloxondosen, die verabreicht wurden, konnten bis zu 72 mg Naloxon betragen [Sykes N.P. "Oral naloxone in opioid-associated constipation", The Lancet 337, 1475 (1991)].

Robinson et al. berichten von einer Studie an 12 Patienten mit opioidinduzierter Obstipation, in der ebenfalls Naloxon oral verabreicht wurde. Die maximal verabreichte Dosis war 12 mg Naloxon. Bei keinem der behandelten Patienten war ein Effekt auf die gastrointestinale Motilität oder Entzugserscheinungen feststellbar [Robinson B.A., Johansson L., Shaw J. "Oral naloxone in opioid-associated constipation", The Lancet 338, 581-582 (1991)].

Bei den zitierten Studien fällt auf, daß die Ergebnisse sehr unterschiedlich ausfallen, und Naloxon vor allem dann wirkt, wenn es in hohen Dosen appliziert wird. In diesem Dosisbereich treten bei vereinzelten Patienten auch schon Entzugssymptome auf. Bei üblichen, einfachen Formulierungen (z.B. Kapseln oder Tropfen) wird der Wirkstoff schnell und nicht modifiziert freigesetzt. Bei Einsatz dieser einfachen Formulierungen wird Naloxon rasch und vollständig im oberen Teil des Gastrointestinaltraktes resorbiert. Durch die resultierenden, relativ hohen Blutkonzentrationen kann es zu unerwünschten Nebenwirkungen kommen. Therapeutische Anwendungen derartiger Naloxon-Formulierungen sind z.B. in EP 0 103 636 (A1) und EP 0 352 361 (A1) beschrieben.

Da die Lähmung aber den ganzen Gastrointestinaltrakt und nicht nur Duodenum und obere Teile des Dünndarms betrifft, kann das Problem der opioidinduzierten Obstipation mit einer derartigen Formulierung (z.B. Tropfen) nicht gelöst werden.

DE 4325465 (A1) schlägt ein Kombinationspräparat aus einem Opioid und einem Opioidantagonisten für die orale Verabreichung vor, wobei das Opioid retardiert, der Opioidantagonist dagegen schnell, d.h. mit geringer oder keiner Retardierung freigesetzt wird. Bezogen auf den Naloxonanteil entspricht dieses Präparat einer nicht modifiziert schnellfreisetzenden Formulierung mit den oben erwähnten Nachteilen. Dieser Weg erhöht nämlich die Gefahr einer unerwünschten systemischen Naloxon-Wirkung, wodurch die schmerzstillende Wirkung des Opioids wieder aufgehoben wird. Daher kann bei Anwendung der Lehre von DE 4325465 (A1) keine vollständige Beseitigung der Nebenwirkungen erreicht werden bzw. können bei den gewählten Dosisbereichen wieder Entzugssymptome beim Patienten auftreten.

Der Erfindung liegt demnach die Aufgabe zugrunde, eine orale galenische Formulierung mit opioidantagonistischer Aktivität zur Verfügung zu stellen, die aufgrund ihrer pharmazeutischtechnologischen Eigenschaften in der Lage ist, die opioidinduzierte Obstipation aufzuheben, ohne dabei zu einer nennenswerten systemischen Verfügbarkeit von Naloxon zu führen und so die Opioidwirkung im ZNS zu antagonisieren.

Die Erfindung beruht auf der Erkenntnis, daß eine wirkungsvolle Antagonisierung der Opioidwirkung auf die oberen und unteren Teile des Gastrointestinaltraktes unter Vermeidung der systemischen Antagonisierung des Opioideffekts nur dann geschehen kann, wenn der Wirkstoff über den gesamten Magen-Darm-Trakt modifiziert freigesetzt wird. Die Steuerung der Freisetzung erfolgt dabei ortsspezifisch über den unterschiedlichen Umgebungs-pH in den jeweiligen Magen- bzw. Darm-Abschnitten, wobei es sich nicht um eine Retardierung im Sinne einer verlangsamten Freisetzung handelt. Hier besteht die Gefahr, daß bei einer bereits vorhandenen Obstipation die Darmpassage der Formulierung verlängert und der Wirkstoff vorzeitig in den oberen Abschnitten des Magen-Darm-Traktes freigesetzt wird, während die unteren Abschnitte nicht versorgt werden.

Die obige Aufgabe wird gelöst durch eine oral verabreichbare, Naloxon, N-Methylnaloxon und/oder N-Methylnaltrexon, oder ein pharmazeutisch verträgliches Salz davon als Wirkstoff enthaltende pharmazeutische Zusammensetzung, wobei die Freisetzung des Wirkstoffs über den gesamten Magen-Darm-Trakt dadurch erreicht wird, daß die in der Zusammensetzung enthaltenen Partikel den Wirkstoff initial und in Abhängigkeit vom Umgebungs-pH freisetzen. Die folgenden Ausführungen über Naloxon gelten in gleicher Weise für N-Methylnaloxon, N-Methylnaltrexon, pharmazeutisch verträglichen Salzen dieser Verbindungen und Mischungen davon.

Bevorzugte Ausführungsformen der Erfindung sind in den abhängigen Ansprüchen wiedergegeben.

Die erfindungsgemäße Naloxon-Formulierung zeichnet sich durch eine gezielte und kontrollierte Wirkstofffreisetzung möglichst gleichmäßig über den gesamten Gastrointestinaltrakt, d.h. vom Magen bis zum Kolon aus, wobei eine schnelle Wirkstofffreisetzung lokal in einzelnen Abschnitten des Magen-Darm-Traktes erfolgt. Da die Wirkstofffreisetzung im Gegensatz zu zeitabhängig kontrolliert freisetzenden Systemen nicht durch die verlangsamte Freisetzung, sondern über die variierenden pH-Verhältnisse im Magen-Darm-Trakt gesteuert wird, führt die opioidinduzierte Obstipation und der damit verbundene verzögerte gastrointestinale Transit der Wirkstoffträger (Pellets o.ä.) nicht zu einer unkontrollierten Freisetzung des Arzneistoffs in Darmabschnitten, in denen nicht freigesetzt werden sollte. Hierdurch ergibt sich bei der erfindungsgemäßen Formulierung der Vorteil, daß eine geringere Einzeldosis eingesetzt werden kann.

In einer bevorzugten Ausführungsform sind die wirkstoffhaltigen Partikel mit einem in Abhängigkeit vom Umgebungs-pH löslichen Überzug versehen. Für einen derartigen Überzug können übliche filmbildende Substanzen mit einer in Abhängigkeit vom Umgebungs-pH unterschiedlichen Löslichkeit verwendet werden. Bevorzugt sind die in der Galenik eingesetzten und bekannten Acrylpolymere der Eudragit®-Reihe, insbesondere Eudragit® L100-55, Eudragit® L100 und Eudragit® S100 (erhältlich von Röhm Pharma GmbH, Weiterstadt, Deutschland). Durch entsprechendes Mischen dieser Substanzen bzw. der mit diesen Substanzen überzogenen Wirkstoffpartikel kann gezielt der gewünschte Freisetzungs-pH eingestellt werden.

Ein weiterer Vorteil ist die mögliche Verringerung der systemischen Belastung und der applizierten Dosis durch das Prinzip des 'Drug Targeting'. Die erfindungsgemäße Arzneiform als Monopräparat ermöglicht ferner die Anwendung bei der Obstipation durch unterschiedlichste Opioide, sie kann aber auch als Kombinationspräparat mit einem bestimmten Opioid, insbesondere Morphin, oder einer oder mehreren Substanzen vom Morphin-Typ eingesetzt werden. Eine Auswahl solcher Opioide umfaßt z.B. Codein, Dihydrocodein, Hydromorphon, Levomethadon, Oxycodon, Pethidin und Propoxyphen und/oder deren Salze. Die Dosierung des Opioids hängt vom Alter, Geschlecht und der Schwere der Erkrankung des Patienten ab, und kann vom behandelnden Arzt aufgrund seiner Fachkenntnisse ohne weiteres eingestellt werden.

Bevorzugte pharmazeutische Arzneiformen enthalten naloxonhaltige Partikel (Pellets, Mikrotabletten oder Granulate) mit unterschiedlichem Lacküberzug. Die Partikel sollten bevorzugt so bemessen sein, daß sie den Pylorus weitgehend unabhängig von der Motilität des Magen-Darm-Traktes passieren. Günstig ist hierfür eine Maximalgröße von ca. 2 mm. Üblicherweise haben die Pellets einen Durchmesser von ca. 1 mm, die beschriebenen Microtabletten von ca. 2 mm. Die mittlere Korngröße des Granulats ist kleiner als ca. 1 mm, bevorzugt ca. 300 bis ca. 600 µm. Die Lacke auf den Partikeln unterscheiden sich durch ihre unterschiedliche Löslichkeitscharakteristik. Die Löslichkeit der Lacke und der damit verbundenen Freisetzung des Arzneistoffs hängt vom lokalen pH-Wert des Magen-Darm-Traktes ab. Eine Mischung verschiedener Partikel mit unterschiedlichem Freisetzungsverhalten macht sich die stark variierenden pH-Verhältnisse im Magen-Darm-Trakt (Magen ca. pH 1,2 Kolon ca. pH 7,0) zunutze.

Bevorzugt enthält die erfindungsgemäße pharmazeutische Zusammensetzung mindestens zwei Typen von Partikeln, die den Wirkstoff jeweils bei einem unterschiedlichen Umgebungs-pH freisetzen. Da beim Menschen die opioidinduzierte Obstipation zu etwa 50% durch eine verzögerte Magenentleerung entsteht und zu jeweils ca. 25% durch die erlahmte propulsive Peristaltik im Dünn- und Dickdarmbereich [Manara L., Bianchetti A. "The central and peripheral influenced of opiods on gastrointestinal propulsion", Ann. Rev. Pharmacol. Toxicol. 25, 249-273 (1985)], ist eine Initialfreisetzung von einer gewissen Wirkstoffmenge empfehlenswert, sobald die Formulierung in den Magen gelangt. Zu diesem Zweck kann der erste Typ von Partikeln so ausgebildet sein, daß der Wirkstoff bereits beim Umgebungs-pH des Magens freigesetzt wird. Der zweite Partikeltyp setzt dann den Wirkstoff beim Umgebungs-pH des unteren Darmtraktes, d.h. einem pH von ca. 7,0 frei. In der Praxis kann der erste Typ von Partikeln den Wirkstoff bereits bei Kontakt mit einem wässrigen Medium pHunabhängiger Weise freisetzen. Diese Initialfreisetzung kann dadurch erreicht-werden, daß der erste Typ von Partikeln mit einem Methylhydroxypropylcellulose und mit gegebenenfalls Polyethylenglykol (z.B. Macrogol® 6000; mittleres Molekulargewicht 6000) als Hilfsstoff enthaltenden Überzug versehen ist, der im wässrigen Medium unabhängig vom pH-Wert löslich ist.

In einer bevorzugten Ausführungsform beträgt das Verhältnis der Partikel vom ersten Typ zu den Partikeln vom zweiten Typ 1:10 bis 10:1, besonders bevorzugt ca. 1:1. Zusätzlich können weitere Typen von Partikeln enthalten sein, die den Wirkstoff bei einem Umgebungs-pH von ca 5,5 bis ca. 6,5 freisetzen.

In einer weiteren bevorzugten Ausführungsform enthält die pharmazeutische Zusammensetzung einen ersten Typ von Partikeln, die den Wirkstoff bei Kontakt mit einem wässrigen Medium pH-unabhängig, und einen zweiten oder mehrere Typen von Partikeln, die den Wirkstoff bei einem Umgebungs-pH von ca. 5,5 bis 7,0 freisetzen.

In der fertigen Arzneiform (z.B. Hartgelatinekapsel) liegt somit eine Mischung von befilmten Partikeln vor, die im Magen, im oberen und unteren Dünndarm und im Kolon den Wirkstoff kontrolliert und modifiziert freisetzt. Die erfindungsgemäße Zusammensetzung kann verabreicht werden, um eine bereits bestehende opioidinduzierte Obstipation zu behandeln. Sie kann aber auch vorsorglich gegeben werden, um das Auftreten einer Obstipation bei der Opioidanalgetikabehandlung von vornherein zu verhindern.

### Beispiele

Die folgenden nicht limitierenden Beispiele geben bevorzugte Ausführungsformen der Erfindung wieder.

### Rezepturbeispiele für Naloxon-Pellets

### Beispiel 1: Naloxon Pellets Typ A (pH-unabhängige Freisetzung im oberen gastrointestinalen (GI)-Trakt)

| Kern | |
|---|---|
| Naloxon-HCl | 2,00 mg |
| Saccharose | 42,00 mg |
| Maisstärke | 12,50 mg |
| Polyvidone | 3,50 mg |

| Filmüberzug | |
|---|---|
| Methylhydroxypropylcellulose | 1,80 mg |
| Macrogol 6000 | 0,18 mg |
| Talkum | 2,02 mg |
| | 64,00 mg |

### Beispiel 2: Naloxon Pellets Typ B (Freisetzung in Darmabschnitten mit einem pH-Milieu von ca. 5,5)

| Kern | |
|---|---|
| Naloxon-HCl | 2,00 mg |
| Saccharose | 42,00 mg |
| Maisstärke | 12,50 mg |
| Polyvidone | 3,50 mg |

| Filmüberzug | |
|---|---|
| Eudragit® L 100-55 | 12,00 mg |
| Triethylcitrat | 1,20 mg |
| Talkum | 3,80 mg |
| | 77,00 mg |

### Beispiel 3: Naloxon Pellets Typ C (Freisetzung in Darmabschnitten mit einem pH-Milieu von ca. 6,0)

| Kern | |
|---|---|
| Naloxon-HCl | 2,00 mg |
| Saccharose | 42,00 mg |
| Maisstärke | 12,50 mg |
| Polyvidone | 3,50 mg |

| Filmüberzug | |
|---|---|
| Eudragit® L 100 | 12,00 mg |
| Triethylcitrat | 1,20 mg |
| Talkum | 3,80 mg |
| | 77,00 mg |

### Beispiel 4: Naloxon Pellets Typ D (Freisetzung in Darmabschnitten mit einem pH-Milieu von ca. 6,5)

| Kern | |
|---|---|
| Naloxon-HCl | 2,00 mg |
| Saccharose | 42,00 mg |
| Maisstärke | 12,50 mg |
| Polyvidone | 3,50 mg |

| Filmüberzug | |
|---|---|
| Eudragit® L 100 | 6,00 mg |
| Eudragit® S 100 | 6,00 mg |
| Triethylcitrat | 1,20 mg |
| Talkum | 3,80 mg |
| | 77,00 mg |

### Beispiel 5: Naloxon Pellets Typ E (Freisetzung in Darmabschnitten mit einem pH-Milieu von ca. 7,0)

| Kern | |
|---|---|
| Naloxon-HCl | 2,00 mg |
| Saccharose | 42,00 mg |
| Maisstärke | 12,50 mg |
| Polyvidone | 3,50 mg |

| Filmüberzug | |
|---|---|
| Eudragit® S 100 | 12,00 mg |
| Triethylcitrat | 1,20 mg |
| Talkum | 3,80 mg |
| | 77,00 mg |

In den Beispielen 1 bis 5 werden die Pelletkerne nach einem bekannten Verfahren (z.B. Extrudierung und anschließende Ausrundung, Aufziehen des Wirkstoffs auf Starterkerne in der Wirbelschicht) hergestellt und anschließend befilmt. Die Abfüllung der Pellets erfolgt in Hartgelatinekapseln.

Die einfachste Pelletkombination enthält die Pellettypen A und E in einem Verhältnis 1 : 10 bzw. 10 : 1, vorzugsweise aber 1 : 1.

Damit der Arzneistoff im Gastrointestinaltrakt gleichmäßiger verteilt wird, können zu der oben erwähnten Mischung die Pellettypen B, C und/oder D gemischt werden. Die Gesamtdosis von Naloxon-HCl in einer Kapsel kann zwischen ca. 1 mg und ca. 30 mg, bevorzugt ca. 1 mg und ca. 10 mg, betragen.

Rezepturbeispiele für Mikrotabletten

### Beispiel 6: Naloxon Mikrotabletten Typ A (pH-unabhängige Freisetzung im oberen GI-Trakt)

| Kern (Durchmesser: 2 mm) | |
|---|---|
| Naloxon-HCl | 0,20 - 0,50 mg |
| Lactose | 6,40 - 6,70 mg |
| mikrokristalline Cellulose | 2,00 mg |
| L-HPC | 1,00 mg |
| Magnesiumstearat | 0,10 mg |

| Filmüberzug | |
|---|---|
| Methylhydroxypropylcellulose | 0,18 mg |
| Macrogol 6000 | 0,018 mg |
| Talkum | 0,202 mg |
| | 10,40 mg |

### Beispiel 7: Naloxon Mikrotabletten Typ B (Freisetzung in Darmabschnitten mit einem pH-Milieu von ca. 5,5)

| Kern (Durchmesser: 2 mm) | |
|---|---|
| Naloxon-HCl | 0,20 - 0,50 mg |
| Lactose | 6,40 -- 6,70 mg |
| mikrokristalline Cellulose | 2,00 mg |
| L-HPC | 1,00 mg |
| Magnesiumstearat | 0,10 mg |

| Filmüberzug | |
|---|---|
| Eudragit® L 100-55 | 0,80 mg |
| Triethylcitrat | 0,08 mg |
| Talkum | 0,12 mg |
| | 11,00 mg |

### Beispiel 8: Naloxon Mikrotabletten Typ C (Freisetzung in Darmabschnitten mit einem pH-Milieu von ca. 6,0)

| Kern (Durchmesser: 2 mm) | |
|---|---|
| Naloxon-HCl | 0,20 - 0,50 mg |
| Lactose | 6,40 -- 6,70 mg |
| mikrokristalline Cellulose | 2,00 mg |
| L-HPC | 1,00 mg |
| Magnesiumstearat | 0,10 mg |

| Filmüberzug | |
|---|---|
| Eudragit® L 100 | 0,80 mg |
| Triethylcitrat | 0,08 mg |
| Talkum | 0,12 mg |
| | 11,00 mg |

### Beispiel 9: Naloxon Mikrotabletten Typ D (Freisetzung in Darmabschnitten mit einem pH-Milieu von ca. 6,5)

| Kern (Durchmesser: 2 mm) | |
|---|---|
| Naloxon-HCl | 0,20 - 0,50 mg |
| Lactose | 6,40 - 6,70 mg |
| mikrokristalline Cellulose | 2,00 mg |
| L-HPC | 1,00 mg |
| Magnesiumstearat | 0,10 mg |

| Filmüberzug | |
|---|---|
| Eudragit® L 100 | 0,40 mg |
| Eudragit® S 100 | 0,40 mg |
| Triethylcitrat | 0,08 mg |
| Talkum | 0,12 mg |
| | 11,00 mg |

### Beispiel 10: Naloxon Mikrotabletten Typ E (Freisetzung in Darmabschnitten mit einem pH-Milieu von ca. 7,0)

| Kern (Durchmesser: 2 mm) | |
|---|---|
| Naloxon-HCl | 0,20 - 0,50 mg |
| Lactose | 6,40 - 6,70 mg |
| mikrokristalline Cellulose | 2,00 mg |
| L-HPC | 1,00 mg |
| Magnesiumstearat | 0,10 mg |

| Filmüberzug | |
|---|---|
| Eudragit® S 100 | 0,80 mg |
| Triethylcitrat | 0,08 mg |
| Talkum | 0,12 mg |
| | 11,00 mg |

Für die Beispiele 6 bis 10 werden die Bestandteile des Tablettenkerns (ohne Magnesiumstearat) gesiebt und in einem geeigneten Freifallmischer 15 min. gemischt. Nach Zugabe des Magnesiumstearates wird weitere 10 Minuten gemischt. Die Masse wird anschließend auf einer Tablettenpresse mit Spezialstempel (Durchmesser 2 mm) zu Mikrotabletten gepreßt. Die erhaltene Mikrotabletten werden in einem geeigneten Gerät befilmt und in Hartgelatinekapseln abgefüllt.

Die einfachste Kombination der Mikrotabletten enthält die Typen A und E in einem Verhältnis 1 : 10 bzw. 10 : 1, vorzugsweise aber 1 : 1.

Damit der Arzneistoff im Gastrointestinaltrakt gleichmäßiger verteilt wird, können zu der oben erwähnten Mischung die Mikrotabletten B, C und/oder D gemischt werden. Die Gesamtdosis von Naloxon-HCl in einer Kapsel kann zwischen ca. 1 mg und ca. 30 mg, bevorzugt ca. 1 mg und ca. 10 mg, betragen.

### Rezepturbeispiele für Granulate

### Beispiel 11: Naloxon Granulat Typ A (pH-unabhängige Freisetzung im oberen GI-Trakt)

| Basisgranulat | |
|---|---|
| Naloxon-HCl | 2,00 - 5,00 g |
| Lactose | 65,00 - 68,00 g |
| mikrokristalline Cellulose | 20,00 g |
| L-HPC | 10,00 g |

| Filmüberzug | |
|---|---|
| Methylhydroxypropylcellulose | 2,70 g |
| Macrogol 6000 | 0,27 g |
| Talkum | 3,03 g |
| | 106,00 g |

### Beispiel 12: Naloxon Granulat Typ B (Freisetzung in Darmabschnitten mit einem pH-Milieu von ca. 5,5)

| Basisgranulat | |
|---|---|
| Naloxon-HCl | 2,00 - 5,00 g |
| Lactose | 65,00 - 68,00 g |
| mikrokristalline Cellulose | 20,00 g |
| L-HPC | 10,00 g |

| Filmüberzug | |
|---|---|
| Eudragit® L 100-55 | 20,00 g |
| Triethylcitrat | 2,00 g |
| Talkum | 3,00 g |
| | 125,00 g |

### Beispiel 13: Naloxon Granulat Typ C (Freisetzung in Darmabschnitten mit einem pH-Milieu von ca. 6,0)

| Basisgranulat | |
|---|---|
| Naloxon-HCl | 2,00 - 5,00 g |
| Lactose | 65,00 - 68,00 g |
| mikrokristalline Cellulose | 20,00 g |
| L-HPC | 10,00 g |

| Filmüberzug | |
|---|---|
| Eudragit® L 100 | 20,00 g |
| Triethylcitrat | 2,00 g |
| Talkum | 3,00 g |
| | 125,00 g |

### Beispiel 14: Naloxon Granulat Typ D (Freisetzung in Darmabschnitten mit einem pH-Milieu von ca. 6,5)

| Basisgranulat | |
|---|---|
| Naloxon-HCl | 2,00 - 5,00 g |
| Lactose | 65,00 - 68,00 g |
| mikrokristalline Cellulose | 20,00 g |
| L-HPC | 10,00 g |

| Filmüberzug | |
|---|---|
| Eudragit® L 100 | 10,00 g |
| Eudragit® S 100 | 10,00 g |
| Triethylcitrat | 2,00 g |
| Talkum | 3,00 g |
| | 125,00 g |

### Beispiel 15: Naloxon Granulat Typ E (Freisetzung in Darmabschnitten mit einem pH-Milieu von ca. 7, 0)

| Basisgranulat | |
|---|---|
| Naloxon-HCl | 2,00 - 5,00 g |
| Lactose | 65,00 - 68,00 g |
| mikrokristalline Cellulose | 20,00 g |
| L-HPC | 10,00 g |

| Filmüberzug | |
|---|---|
| Eudragit® S 100 | 20,00 g |
| Triethylcitrat | 2,00 g |
| Talkum | 3,00 g |
| | 125,00 g |

Die Bestandteile des Basisgranulates gemäß den Beispielen 11 bis 15 werden gesiebt und in einem geeigneten Mischer mit Granulierflüssigkeit befeuchtet und granuliert. Das Granulat wird anschließend in der Wirbelschicht getrocknet und gesiebt, so daß ein Granulat mit einer mittleren Korngröße von vorzugsweise 300 bis 600 µm erhalten wird. Die Granulate werden in einem geeigneten Gerät befilmt. Die Gesamtdosis des Wirkstoffs in einer Granulatformulierung kann zwischen ca. 1 mg und ca. 30 mg, bevorzugt ca. 1 mg und ca. 10 mg, betragen.

Grundsätzlich ist es möglich, verschiedene Granulate in gewünschten Verhältnissen miteinander zu mischen.

Die befilmten Granulate können wie folgt weiter verarbeitet werden:
- Abfüllung in Hartgelatinekapseln
- Verpressung zu Tabletten nach Zumischen geeigneter Tablettierhilfsstoffe (z. B. mikrokristalline Cellulose, Magnesiumstearat)
- Abfüllung in Sachetbeutel nach Zumischen weiterer Hilfsstoffe ( z. B. Saccharose, Nadioctylsulfosuccinat, Xanthan Gum, Aromastoffe).

## Patentansprüche

1. Oral verabreichbare, pharmazeutische Zusammensetzung, die einen Opioidantagonisten, ausgewählt aus Naloxon, N-Methylnaloxon und N-Methylnaltrexon, oder ein pharmazeutisch verträgliches Salz davon, als Wirkstoff enthält, mit Freisetzung des Wirkstoffs über den gesamten Magen-Darm-Trakt
**dadurch gekennzeichnet, daß**
die Zusammensetzung wirkstoffhaltige Partikel enthält, die den Wirkstoff in Abhängigkeit vom Umgebungs-pH des Magen-Darm-Traktes freisetzen.

2. Pharmazeutische Zusammensetzung nach Anspruch 1, **dadurch gekennzeichnet, daß** die Partikel mit einem in Abhängigkeit vom Umgebungs-pH löslichen Überzug versehen sind.

3. Pharmazeutische Zusammensetzung nach Anspruch 2, **dadurch gekennzeichnet, daß** der Überzug eine oder mehrere filmbildende Substanzen mit einer in Abhängigkeit vom Umgebungs-pH unterschiedlichen Löslichkeit, ausgewählt aus Eudragit® L100-55, Eudragit® L100 und Eudragit® S100, enthält.

4. Pharmazeutische Zusammensetzung nach einem der Ansprüche 1 bis 3, **dadurch gekennzeichnet, daß** die Partikel als Pellets, Mikrotabletten oder Granulate mit einem mittleren Durchmesser von nicht größer als 2 mm ausgebildet sind.

5. Pharmazeutische Zusammensetzung nach einem der Ansprüche 1 bis 4, **dadurch gekennzeichnet, daß** die Zusammensetzung mindestens zwei Typen von Partikeln enthält, die den Wirkstoff jeweils bei einem unterschiedlichen Umgebungs-pH freisetzen.

6. Pharmazeutische Zusammensetzung nach Anspruch 1, **dadurch gekennzeichnet, daß** sie einen ersten Typ von Partikeln, die den Wirkstoff beim Umgebungs-pH des Magens oder bei Kontakt mit einem wässrigen Medium pH-unabhängig freisetzen, und einen zweiten Typ von Partikeln, die den Wirkstoff beim Umgebungs-pH des unteren Darmtraktes freisetzen, enthält.

7. Pharmazeutische Zusammensetzung nach Anspruch 6, **dadurch gekennzeichnet, daß** der erste Typ von Partikeln den Wirkstoff bei Kontakt mit einem wässrigen Medium pH-unabhängig freisetzt.

8. Pharmazeutische Zusammensetzung nach Anspruch 7, **dadurch gekennzeichnet, daß** der erste Typ von Partikeln mit einem Methylhydroxypropylcellulose und gegebenenfalls Polyethylenglykol als Hilfsstoff enthaltenden Überzug versehen ist.

9. Pharmazeutische Zusammensetzung nach Anspruch 6, **dadurch gekennzeichnet, daß** das Verhältnis der Partikel vom ersten Typ zu den Partikeln vom zweiten Typ 1:10 bis 10:1 beträgt.

10. Pharmazeutische Zusammensetzung nach Anspruch 6, **dadurch gekennzeichnet, daß** das Verhältnis der Partikel vom ersten Typ zu den Partikeln vom zweiten Typ ca. 1:1 beträgt.

11. Pharmazeutische Zusammensetzung nach einem der Ansprüche 6 bis 10, **dadurch gekennzeichnet, daß** sie einen ersten Typ von Partikeln, die den Wirkstoff bei Kontakt mit einem wässrigen Medium pH-unabhängig, und einen zweiten oder mehrere Typen von Partikeln, die den Wirkstoff bei einem Umgebungs-pH von ca. 5,5 bis 7,0 freisetzen, enthält.

12. Pharmazeutische Zusammensetzung nach einem der Ansprüche 1 bis 11 zur Verwendung bei der Therapie und/oder Prophylaxe von opioidinduzierter Obstipation.

13. Pharmazeutische Zusammensetzung nach Anspruch 12, **dadurch gekennzeichnet, daß** die Obstipation durch Morphin oder Substanzen vom Morphin-Typ verursacht wird.

14. Pharmazeutische Zusammensetzung nach einem der Ansprüche 1 bis 13, **dadurch gekennzeichnet, daß** die Zusammen-setzung zusätzlich Morphin oder Substanzen vom Morphin-Typ enthält.

15. Pharmazeutische Zusammensetzung nach Anspruch 14, **dadurch gekennzeichnet, daß** die Substanzen vom Morphin-Typ ausgewählt sind aus Codein, Dihydrododein, Hydromorphon, Levomethadon, Oxycodon, Pethidin und Propoxyphen.

16. Pharmazeutische Zusammensetzung nach einem der Ansprüche 1 bis 15, **dadurch gekennzeichnet, daß** die Zusammen-setzung zusammen mit üblichen pharmazeutisch verträglichen Hilfsstoffen als Tabletten-, Kapsel- oder Granulatformulierung vorliegt.

17. Pharmazeutische Zusammensetzung nach Anspruch 16, **dadurch gekennzeichnet, daß** die Zusammensetzung zusammen mit üblichen pharmazeutisch verträglichen Hilfsstoffen als Tabletten-, Kapsel- oder Granulatformulierung als Einheitsdosis-formulierung enthaltend ca. 1 mg bis ca. 30 mg Opioidantagonist Wirkstoff vorliegt.

18. Pharmazeutische Zusammensetzung nach Anspruch 17, **dadurch gekennzeichnet, daß** die Zusammensetzung, zusammen mit üblichen pharmazeutisch verträglichen Hilfsstoffen als Tabletten-, Kapsel- oder Granulatformulierung als Einheitsdosis-formulierung enthaltend ca. 1 mg bis ca. 10 mg Opioidantagonist Wirkstoff vorliegt.

19. Oral verabreichbare, pharmazeutische Zusammensetzung, die einen Opioidantagonisten, ausgewählt aus Naloxon, N-Methylnaloxon und N-Methylnaltrexon, oder ein pharmazeutisch verträgliches Salz davon, als Wirkstoff enthält, mit Freisetzung des Wirkstoffs über den gesamten Magen-Darm-Trakt
**dadurch gekennzeichnet, daß**
die Steuerung der Freisetzung ortsspezifisch über H den unterschiedlichen Umgebungs-pH in den jeweiligen Magen-bzw. Darm-Abschnitten erfolgt.

## Claims

1. Orally administrable pharmaceutical composition which comprises as active ingredient an opioid antagonist selected from naloxone, N-methylnaloxone and N-methylnaltrexone, or a pharmaceutically acceptable salt thereof, with release of the active ingredient over the entire gastro-intestinal tract, **characterized in that** the composition comprises particles comprising active ingredient, which particles release the active ingredient as a function of the ambient pH of the gastro-intestinal tract.

2. Pharmaceutical composition according to claim 1, **characterized in that** the particles are provided with a coating soluble as a function of the ambient pH.

3. Pharmaceutical composition according to claim 2, **characterized in that** the coating comprises one or more film-forming substances with a solubility differing as a function of the ambient pH, selected from Eudragit® L100-55, Eudragit® L100 and Eudragit® S100.

4. Pharmaceutical composition according to any one of claims 1 to 3, **characterized in that** the particles are formed as pellets, microtablets or granulates having an average diameter not greater than 2 mm.

5. Pharmaceutical composition according to any one of claims 1 to 4, **characterized in that** the composition comprises at least two types of particles, each of which releases the active ingredient at a different ambient pH.

6. Pharmaceutical composition according to claim 1, **characterized in that** it comprises a first type of particles, which releases the active ingredient at the ambient pH of the stomach or in a pH-independent manner on contact with an aqueous medium, and a second type of particles which releases the active ingredient at the ambient pH of the lower intestinal tract.

7. Pharmaceutical composition according to claim 6, **characterized in that** the first type of particles releases the active ingredient in a pH-independent manner on contact with an aqueous medium.

8. Pharmaceutical composition according to claim 7, **characterized in that** the first type of particles is provided with a coating comprising methylhydroxypropyl cellulose and, optionally, polyethylene glycol as adjuvant.

9. Pharmaceutical composition according to claim 6, **characterized in that** the ratio of the particles of the first type to the particles of the second type is from 1:10 to 10:1.

10. Pharmaceutical composition according to claim 6, **characterized in that** the ratio of the particles of the first type to the particles of the second type is approximately 1:1.

11. Pharmaceutical composition according to any one of claims 6 to 10, **characterized in that** it comprises a first type of particles, which releases the active ingredient in a pH independent manner on contact with an aqueous medium, and a second type or further types of particles which release(s) the active ingredient at an ambient pH of approximately from 5.5 to 7.0.

12. Pharmaceutical composition according to any one of claims 1 to 11 for use in the treatment and/or prevention of opioid-induced constipation.

13. Pharmaceutical composition according to claim 12, **characterized in that** the constipation is caused by morphine or substances of the morphine type.

14. Pharmaceutical composition according to any one of claims 1 to 13, **characterized in that** the composition additionally comprises morphine or substances of the morphine type.

15. Pharmaceutical composition according to claim 14, **characterized in that** the substances of the morphine type are selected from codeine, dihydrocodeine, hydromorphone, levomethadone, oxycodone, pethidine and propoxyphene.

16. Pharmaceutical composition according to any one of claims 1 to 15, **characterized in that** the composition together with customary pharmaceutically acceptable adjuvants is in the form of a tablet, capsule or granulate formulation.

17. Pharmaceutical composition according to claim 16, **characterized in that** the composition together with customary pharmaceutically acceptable adjuvants is in the form of a tablet, capsule or granulate formulation as a unit dose formulation containing from approximately 1 mg to approximately 30 mg of opioid antagonist active ingredient.

18. Pharmaceutical composition according to claim 17, **characterized in that** the composition together with customary pharmaceutically acceptable adjuvants is in the form of a tablet, capsule or granulate formulation as a unit dose formulation containing from approximately 1 mg to approximately 10 mg of opioid antagonist active ingredient.

19. Orally administrable pharmaceutical composition which comprises as active ingredient an opioid antagonist selected from naloxone, N-methylnaloxone and N-methylnaltrexone, or a pharmaceutically acceptable salt thereof, with release of the active ingredient over the entire gastro-intestinal tract, **characterized in that** the control of the release is effected location-specifically by way of the differing ambient pH in the stomach and/or intestine segments in question.

## Revendications

1. Composition pharmaceutique administrable par voie orale, qui contient comme substance active un antagoniste d'opioïde choisi parmi la naloxone, la N-méthylnaloxone et la N-méthylnaltrexone, ou un sel pharmaceutiquement acceptable de celui-ci, à libération de la substance active sur tout le tractus gastro-intestinal, **caractérisée en ce que** la composition contient des particules contenant de la substance active, qui libèrent la substance active en fonction du pH de l'environnement du tractus gastro-intestinal.

2. Composition pharmaceutique selon la revendication 1, **caractérisée en ce que** les particules sont munies d'un enrobage soluble en fonction du pH de l'environnement.

3. Composition pharmaceutique selon la revendication 2, **caractérisée en ce que** l'enrobage contient une ou plusieurs substances filmogènes ayant une solubilité différente en fonction du pH de l'environnement, choisies parmi l'Eudragit® L100-55, l'Eudragit® L100 et l'Eudragit® S100.

4. Composition pharmaceutique selon l'une quelconque des revendications 1 à 3, **caractérisée en ce que** les particules sont constituées de pastilles, microcomprimés ou granulés ayant un diamètre moyen n'excédant pas 2 mm.

5. Composition pharmaceutique selon l'une quelconque des revendications 1 à 4, **caractérisée en ce que** la composition contient au moins deux types de particules qui libèrent la substance active chacune à un pH différent de l'environnement.

6. Composition pharmaceutique selon la revendication 4, **caractérisée en ce qu'**elle contient un premier type de particules qui libèrent la substance active au pH de l'environnement de l'estomac ou au contact avec un milieu aqueux indépendamment du pH, et un second type de particules qui libèrent la substance active au pH de l'environnement du tractus intestinal inférieur.

7. Composition pharmaceutique selon la revendication 6, **caractérisée en ce que** le premier type de particules de la substance active libère la substance active au contact avec un milieu aqueux, en fonction du pH.

8. Composition pharmaceutique selon la revendication 7, **caractérisée en ce que** le premier type de particules est muni d'un enrobage contenant de la méthylhydroxypropylcellulose et éventuellement du polyéthylèneglycol en tant qu'adjuvant.

9. Composition pharmaceutique selon la revendication 6, **caractérisée en ce que** le rapport des particules du premier type aux particules du second type va de 1:10 à 10:1.

10. Composition pharmaceutique selon la revendication 6, **caractérisée en ce que** le rapport des particules du premier type aux particules du second type est d'environ 1:1.

11. Composition pharmaceutique selon l'une quelconque des revendications 6 à 10, **caractérisée en ce qu'**elle contient un premier type de particules qui libèrent la substance active au contact avec un milieu aqueux indépendamment du pH, et un second type ou plusieurs types de particules qui libèrent la substance active à un pH de l'environnement d'environ 5,5 à 7,0.

12. Composition pharmaceutique selon l'une quelconque des revendications 1 à 11, pour utilisation dans la thérapie et/ou la prophylaxie de la constipation induite par des opioïdes.

13. Composition pharmaceutique selon la revendication 12, **caractérisée en ce que** la constipation est provoquée par la morphine ou des substances du type de la morphine.

14. Composition pharmaceutique selon l'une quelconque des revendications 1 à 13, **caractérisée en ce que** la composition contient en outre de la morphine ou des substances du type de la morphine.

15. Composition pharmaceutique selon la revendication 14, **caractérisée en ce que** les substances du type de la morphine sont choisies parmi la codéine, la dihydrocodéine, l'hydromorphone, la lévométhadone, l'oxycodone, la péthidine et le propoxyphène.

16. Composition pharmaceutique selon l'une quelconque des revendications 1 à 15, **caractérisée en ce que** la composition, conjointement avec des adjuvants usuels, pharmaceutiquement acceptables, se trouve sous forme de comprimé, gélule ou granulé.

17. Composition pharmaceutique selon la revendication 16, **caractérisée en ce que** la composition, conjointement avec des adjuvants usuels, pharmaceutiquement acceptables, sous forme de comprimé, gélule ou granulé, se trouve sous forme de dose unitaire contenant d'environ 1 mg à environ 30 mg de substance active antagoniste d'opioïde.

18. Composition pharmaceutique selon la revendication 17, **caractérisée en ce que** la composition, conjointement avec des adjuvants usuels, pharmaceutiquement acceptables, sous forme de comprimé, gélule ou granulé, se trouve sous forme de dose unitaire contenant d'environ 1 mg à environ 10 mg de substance active antagoniste d'opioïde.

19. Composition pharmaceutique administrable par voie orale, qui contient un antagoniste d'opioïde, choisi parmi la naloxone, la N-méthylnaloxone et la N-méthylnaltrexone, ou un sel pharmaceutiquement acceptable de celui-ci, à libération de la substance active sur tout le tractus gastro-intestinal, **caractérisée en ce que** la commande de la libération s'effectue de façon spécifique de site, par le pH variable de l'environnement dans les segments respectifs de l'estomac et, respectivement, de l'intestin.
